# FASCICULE DE BREVET EUROPEEN

(11) **EP 1 457 201 B1**
(45) Date de publication et mention de la délivrance du brevet: **11.04.2007**
(21) Numéro de dépôt: 04290593.5
(22) Date de dépôt: 04.03.2004
(51) Int. Cl.: A61K 8/37, A61K 8/92, A61Q 1/00, A61Q 1/06, A61Q 1/02

(54) **Composition cosmétique contenant un polyester de triglycéride d'acides carboxyliques hydroxyles et une huile de masse molaire allant de 650 à 1000 g/mol.**
Kosmetisches Mittel enthaltend ein Polyester von hydroxylierten Carbonsäuren Triglycerid und ein Öl mit einer Molmasse von 650 bis 1000 g/mol
Cosmetic composition containing a polyester of a hydrocarboxylated carboxylic acid triglyceride and an oil with a molar mass from 650 to 1000 g/ml

(30) Priorité: 12.03.2003 FR 0303078
(43) Date de publication de la demande: 15.09.2004
(73) Titulaire: L'ORÉAL, 75008 Paris (FR)
(72) Inventeur: Blin, Xavier, 75015 Paris (FR); Filippi, Vanina, 75015 Paris (FR)
(74) Mandataire: Boulard, Denis

(56) Documents cités:
- EP-A- 1 097 699
- EP-A- 1 112 734
- FR-A- 2 829 924
- US-A- 5 786 389
- US-A1- 2003 007 950
- US-B1- 6 342 527
- O'LENICK JR. ET AL.: "Castor Polyesters for Personal care" COSMETICS TOILETRIES, vol. 117, no. 6, juin 2002 (2002-06), pages 59-64, XP0008024748

## Description

La présente invention se rapporte à une composition cosmétique de maquillage et/ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable contenant un ester aliphatique d'ester particulier. Cette composition possède des propriétés cosmétiques remarquables et confère en particulier au maquillage ou au soin des propriétés de propriétés de glissant, brillance, confort, netteté des contours, non migration, d'intensité de la couleur améliorée et/ou de tenue de la couleur après épreuves améliorée.

La composition de l'invention peut en particulier constituer un produit de maquillage du corps, des lèvres ou des phanères d'êtres humains ayant en particulier des propriétés de soin et/ou de traitement non thérapeutique. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

Il existe de nombreuses compositions cosmétiques pour lesquelles les propriétés de brillance du film déposé, après application sur les matières kératiniques (peau, lèvres, phanères), sont souhaitables. On peut citer par exemple les rouges à lèvres, les vernis à ongles ou encore certains produits capillaires.

Dans cette optique, le formulateur dispose de plusieurs types de matières premières et notamment des lanolines utilisées en association avec des huiles dites « brillantes », comme a) les polybutènes qui ont une viscosité élevée, b) des esters d'acide ou d'alcool gras dont le nombre de carbone est élevé (typiquement supérieur à 16), c) certaines huiles végétales, d) des esters résultants de l'estérification partielle ou totale d'un composé aliphatique hydroxylé avec un acide aromatique, comme décrit dans la demande de brevet EP 1 097 699 et e) des polyesters obtenus par réaction séquencée de l'huile de ricin avec l'acide isostéarique puis avec l'acide succinique décrits dans le brevet US 6 342 527.

L'invention a pour objet une composition cosmétique brillante de soin et/ou de maquillage des matières kératiniques et notamment de la peau et/ou des lèvres et/ou des phanères qui présente des propriétés améliorées par rapport aux compositions cosmétiques de l'art antérieur, notamment dont le dépôt sur les matières kératiniques est plus net au contour et dont la tenue de la couleur est améliorée.

Le demandeur a trouvé de façon surprenante que l'utilisation i) d'au moins un polyester résultant de l'estérification d'au moins un triglycéride d'acide(s) aliphatique(s) hydroxylé(s) par un acide monocarboxylique aliphatique et par un acide dicarboxylique aliphatique, associé à ii) au moins une (à savoir une ou plusieurs) huile de masse molaire allant de 650 à 10000 g/mol permet l'obtention d'une composition cosmétique brillante à l'application et dans le temps, présentant de bonnes propriétés à l'application, de bonnes propriétés d'étalement, de tenue de la couleur après épreuves, de confort (non tiraillement, non dessèchement), de non migration et/ou dont les contours du dépôt sur les matières kératiniques sont nets et/ou dont l'intensité de la couleur est améliorée.

Dans la suite du texte, l'huile de masse molaire allant de 650 à 10 000 g/mol sera appelée huile de masse molaire élevée.

L'invention a donc pour objet une composition cosmétique de soin et/ou de maquillage des matières kératiniques comprenant un milieu cosmétiquement acceptable contenant i) au moins un polyester résultant de l'estérification d'au moins un triglycéride d'acide(s) carboxylique(s) hydroxylé(s) par un acide monocarboxylique aliphatique et par un acide dicarboxylique aliphatique, ii) au moins une huile de masse molaire élevée allant de 650 à 10000 g/mol, et iii) au moins une matière colorante.

L'invention a également pour objet un procédé cosmétique pour conférer à un film de composition cosmétique de la brillance à l'application et dans le temps, de bonnes propriétés à l'application, de bonnes propriétés d'étalement, de tenue de la couleur après épreuves, de confort (non tiraillement, non dessèchement), de non migration et/ou dont les contours du dépôt sur les matières kératiniques sont nets et/ou dont l'intensité de la couleur est améliorée, ledit procédé consistant à introduire dans ladite composition i) au moins un polyester résultant de l'estérification d'au moins un triglycéride d'acide(s) carboxylique(s) hydroxylé(s) par un acide monocarboxylique aliphatique et par un acide dicarboxylique aliphatique, ii) au moins une huile de masse molaire élevée allant de 650 à 10000 g/mol, et iii) au moins une matière colorante.

L'invention a encore pour objet l'utilisation de l'association i) d'au moins un polyester résultant de l'estérification d'au moins un triglycéride d'acide(s) carboxylique(s) hydroxylé(s) par un acide monocarboxylique aliphatique et par un acide dicarboxylique aliphatique et ii) d'au moins une huile de masse molaire élevée allant de 650 à 10000 g/mol dans une composition physiologiquement acceptable contenant au moins une matière colorante, pour conférer à ladite composition de la brillance à l'application et dans le temps, de bonnes propriétés à l'application, de bonnes propriétés d'étalement, de tenue de la couleur après épreuves, de confort (non tiraillement, non dessèchement), de non migration et/ou d'intensité de la couleur améliorée et/ou de tenue de la couleur après épreuves améliorée.

La composition selon l'invention est avantageusement exempte de lanoline ou de dérivés de lanoline.

On entend par dérivés de lanoline, notamment la lanoline liquide, la lanoline réduite, la lanoline purifiée par adsorption, l'acétate de lanoline, la cire de lanoline, par exemple la cire de lanoline oxypropylénée (5 OP) commercialisée sous la référence EMERY 1695 par COGNIS, le lanolate d'isopropyle, l'acétate de lanoline liquide, l'hydroxylanoline, la polyoxyéthylène-lanoline, l'acide gras de lanoline, l'acide gras de lanoline dure, les esters de cholestéryle d'acide gras de lanoline, l'alcool de lanoline, l'acétate de l'alcool de lanoline, et autres.

La composition selon l'invention contient avantageusement au moins un composé pâteux différent des dérivés de la lanoline.

### Polyester de triglycéride d'acide(s) aliphatique(s) hydroxylé(s)

La composition comprend au moins un polyester résultant de l'estérification d'au moins un triglycéride d'acide(s) carboxylique(s) hydroxylé(s) par un acide monocarboxylique aliphatique et par un acide dicarboxylique aliphatique, éventuellement insaturé.

Par acide carboxylique hydroxylé, on entend un acide hydroxy-carboxylique aliphatique. Par triglycéride d'acide(s) carboxylique(s) hydroxylé(s), on entend un glycérol substitué par trois restes d'acides carboxyliques hydroxylés, lesquelles peuvent être identiques ou différents. Par exemple, un triglycéride obtenu par réaction d'un équivalent de glycérol et trois équivalents d'un acide carboxylique hydroxylé sera dénommé "triglycéride d'acide carboxylique hydroxylé". Un triglycéride obtenu par réaction d'un équivalent de glycérol avec trois équivalents d'un mélange d'au moins deux acides carboxyliques hydroxylés différents sera dénommé "triglycéride d'acide(s) carboxylique(s) hydroxylé(s)".

De préférence, le polyester selon l'invention présente une viscosité à 25°C supérieure à 500 cP (50 Pa.s), de préférence allant de 900 à 10 000 cP (90 à 1 000 Pa.s) et mieux de 950 à 5 000 cP (95 à 500 Pa.s), mesurée en particulier avec un viscosimètre type Brookfield RV ou un viscosimètre Brookfield "DV-II+" de type LV équipé d'une aiguille n° 1 tournant entre 0,5 et 10 tr/min. La mesure de la viscosité est prise lorsque la valeur de la mesure est stabilisée, en général au bout de 10 minutes.

De préférence, le polyester selon l'invention a un indice de réfraction supérieur ou égal à 1,47 et notamment allant de 1,47 à 1,55 (l'indice de réfraction étant défini pour la raie D du sodium).

Selon un mode de mise en oeuvre, le polyester est avantageusement obtenu par deux réactions d'estérification d'un triglycéride d'acide(s) carboxylique(s) hydroxylé(s) : une estérification par un acide monocarboxylique aliphatique et une estérification par un acide dicarboxylique aliphatique.

Dans ce mode de mise en oeuvre, le polyester est avantageusement obtenu par
a) l'estérification par un acide monocarboxylique aliphatique d'une partie des fonctions hydroxyles d'un triglycéride d'acide(s) carboxylique(s) hydroxylé(s), et par
b) l'estérification par un acide dicarboxylique aliphatique des fonctions hydroxyles restantes dudit triglycéride d'acide(s) carboxylique(s) hydroxylé(s) estérifié par ledit acide monocarboxylique aliphatique.
L'estérification par un acide monocarboxylique est de préférence conduite avant l'estérification par un acide dicarboxylique aliphatique.

Le ou les acides carboxyliques hydroxylés (précurseur(s) du triglycéride d'acide(s) carboxylique(s) hydroxylé(s)) sont de préférence choisis parmi les acides carboxyliques aliphatiques hydroxylés comprenant de 6 à 40 atomes de carbone, de préférence de 10 à 34 atomes de carbone et mieux de 12 à 28 atomes de carbone, de préférence de 16 à 20 atomes de carbone, de préférence de 18 atomes de carbone.

Le ou les acides carboxyliques hydroxylés sont de préférence choisis parmi les acides gras saturés ou insaturés.

Le ou les acides carboxyliques hydroxylés peuvent être choisis parmi :
i) les acides monocarboxyliques aliphatiques mono hydroxylés linéaires saturés de formule :
   (1) 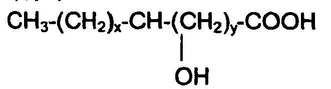 avec 3 ≤ x + y ≤ 37
   ou (2)

      HO-CH₂-(CH₂)ₓ-COOH

      avec 4 ≤ x ≤ 38 ;
ii) les acides monocarboxyliques aliphatiques mono hydroxylés ramifiés saturés de formule :
   (3) 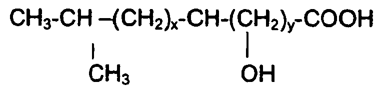 avec 1 ≤ x + y ≤ 35 Ou (3') l'acide 2-éthyl 3-hydroxy caprylique de formule : 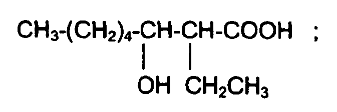
iii) les acides monocarboxyliques aliphatiques mono hydroxylés insaturés de formule :
   (4) 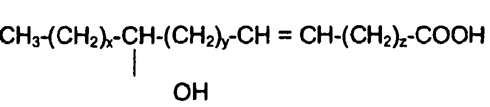 avec 1 ≤ x + y + z ≤ 35
   ou (5) 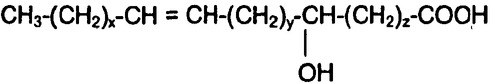 avec 1 ≤ x + y + z ≤ 35
   ou (6)

      HOCH₂-(CH₂)ₓ-CH = CH-(CH₂)_{y}-COOH

      avec 2 ≤ x + y ≤ 36 ;
iv) les acides monocarboxyliques aliphatiques poly hvdroxvlés saturés de formule :
   (7) 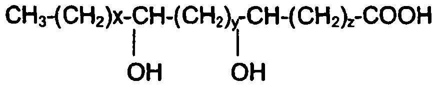 avec 2 ≤ x + y + z ≤ < 36 ; et les acides monocarboxyliques aliphatiques poly hydroxylés insaturés correspondants,
v) les polyacides aliphatiques mono hydroxylés saturés de formule:
   (8) 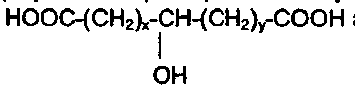 avec 3 ≤ x + y ≤ 37 ; et les polyacides aliphatiques mono hydroxylés insaturés correspondants,
vi) les polyacides aliphatiques poly hydroxylés saturés ou insaturés;
et leurs mélanges.

De façon préférentielle, le ou les acides carboxyliques hydroxylés sont choisis parmi :
- l'acide 12-hydroxy stéarique ; l'acide α-hydroxy octadécanoïque ; l'acide hydroxy 14-eicosènoïque
- l'acide leucinique ou l'acide 2-éthyl 3-hydroxy caprylique ;
- l'acide ricinoléïque ;
- l'acide 3-hydroxy 4-hexanoïque ou l'acide oxynervonique ;
- l'acide 16-hydroxy 6-hexadécènoïque ;
- l'acide 9, 10-dihydroxy octadécanoïque, l'acide 9, 12-dihydroxy octadécanoïque, l'acide aleuritique, l'acide 9, 10, 12-trihydroxy octadécanoïque, l'acide hexahydroxy octadécanoïque ou l'acide octahydroxy octadécanoïque ;
et leurs mélanges.

Le ou les acides carboxyliques hydroxylés sont de préférence choisis parmi les acides gras insaturés comprenant 16 à 20 atomes de carbone, de préférence 18 atomes de carbone.

Le triglycéride est de préférence le triglycéride de l'acide ricinoléïque. Ce triglycéride se trouve en grande quantité à l'état naturel dans l'huile de ricin.

Le triglycéride d'acide(s) carboxylique(s) hydroxylé(s) est avantageusement choisi parmi les triglycérides d'acides hydroxylés tels que lesdits acides hydroxylés comprennent de 6 à 40 atomes de carbone, de préférence de 10 à 34 atomes de carbone et mieux de 12 à 28 atomes de carbone, de préférence de 16 à 20 atomes de carbone, de préférence 18 atomes de carbone.

L'acide monocarboxylique aliphatique peut être un acide gras aliphatique saturé ou insaturé, comme l'acide isostéarique.

L'acide dicarboxylique aliphatique comprend de préférence de 3 à 10 atomes de carbones, de préférence de 3 à 6 atomes de carbone, de préférence de 3 à 5 atomes de carbone. Selon un mode de mise en oeuvre, l'acide dicarboxylique aliphatique correspond à la formule HOOC-(CH₂)ₙ-COOH telle que n=1 à 4.

L'acide dicarboxylique aliphatique est de préférence l'acide succinique, correspondant à la formule précédente dans laquelle n = 2.

Selon un mode de mise en oeuvre préféré, le polyester répond à la formule

T₂O-(OC-D-CO-O-T₁-O)x-OC-D-CO-OT₂ (I)

dans laquelle
T₂-OH représente un triglycéride d'acide(s) carboxylique(s) hydroxylé(s), ledit triglycéride ayant été estérifié par deux molécules d'un acide monocarboxylique aliphatique, et ledit triglycéride comprenant une seule fonction hydroxyle libre ;
OH-T₁-OH représente un triglycéride d'acide(s) carboxylique(s) hydroxylé(s), ledit triglycéride ayant été estérifié par une molécule d'acide monocarboxylique aliphatique, et ledit triglycéride comprenant deux fonctions hydroxyles libres ;
HOOC-D-COOH représente ledit acide dicarboxylique et
x est compris entre 1 et 50, de préférence entre 1 et 10, de préférence encore entre 2 et 6.

x peut être égal à 3, 4, 5, 6, 7, 8, 9 ou 10.

Le polyester est avantageusement un des polyesters décrits dans le brevet US 6 342 527 dont le contenu est inclus par référence dans la présente demande. Le polyester en question répond à la formule (I) ci-dessus dans laquelle
T₂O-représente 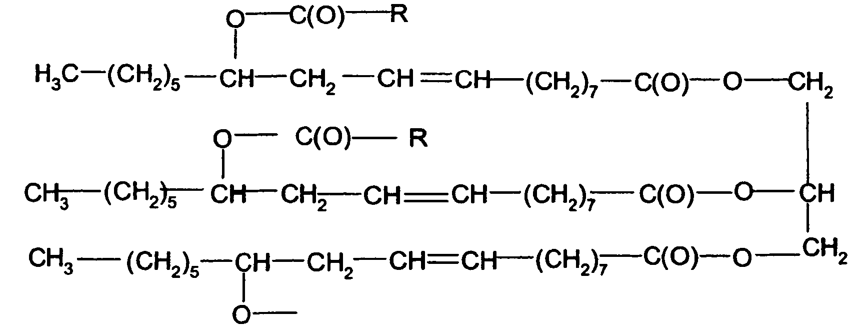 -OT₁O- représente

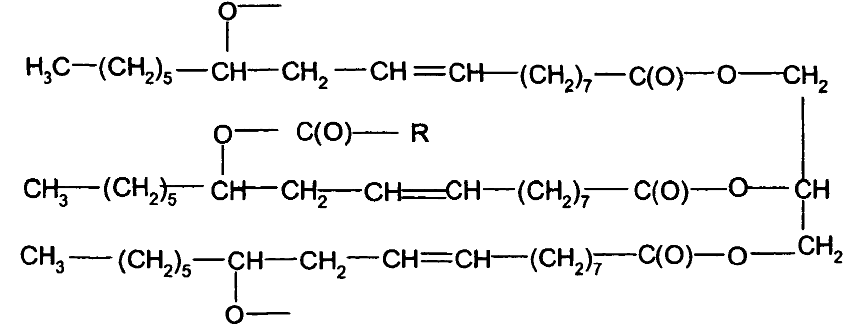

Dans ces formules, R représente un groupe alkyle ou alkylène comprenant 5 à 33 atomes de carbone,

R représente de préférence un alkyle ayant 7 à 17 atomes de carbones ou un alkylène ayant de 11 à 21 atomes de carbones.

Le polyester de la composition de l'invention peut représenter de 0,1 à 99,9 % du poids total de la composition, de préférence de 1 à 99 %, mieux de 1 à 80 %, encore mieux de 10 à 40%, encore mieux de 15 à 25% encore mieux de 20 à 25 %, et de façon générale, être présent en une quantité suffisante pour conférer à la composition des propriétés de brillance, de stabilité, de tenue de la couleur dans le temps, de tenue de la brillance, de confort, de non migration et/ou de netteté des contours du dépôt.

### Huile de masse moléculaire élevée

La composition selon l'invention contient également une huile de masse molaire élevée allant de 650 à 10000 g/mol. Par « huile », on entend un composé non aqueux, non miscible à l'eau, liquide à température ambiante (25°C) et pression atmosphérique (760 mm de Hg).

L'huile utilisée dans la composition selon la présente invention a une masse molaire allant de 650 à 10000 g/mol, et de préférence entre 750 et 7500 g/mol.

En effet, les huiles de masse molaire trop faible associées au polyester de triglycéride d'acide(s) carboxylique(s) hydroxylé(s) dans la composition selon l'invention, conduisent à des compositions qui ne sont pas assez brillantes ; les huiles ayant une masse molaire trop élevée donnent, elles, des compositions jugées trop collantes.

Ainsi, le triglycéride d'acides caprique/caprylique (tel que celui commercialisé ou fabriqué sous la référence ESTOL 3603 MCT OIL par la société Uniquema), qui a une masse molaire égale à 494 g/mol, conduit à des compositions ayant de moins bonnes propriétés cosmétiques que celles de la composition de l'invention.

L'huile de masse molaire allant de 650 à 10000 g/mol utilisable dans la présente invention peut être choisie parmi :
- les polymères lipophiles tels que :
   - les polybutylènes tels que L'INDOPOL H-100 (de masse molaire ou MM=965 g/mol), L'INDOPOL H-300 (MM=1340 g/mol), L'INDOPOL H-1500 (MM=2160g/mol) commercialisés ou fabriqués par la société AMOCO,
   - les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
   - les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,
   - les copolymères de la vinylpyrrolidone tels que : le copolymère vinylpyrrolidone/1-héxadécène, ANTARON V-216 commercialisé ou fabriqué par la société ISP (MM=7300 g/mol),
- les esters tels que :
   - les esters d'acides gras linéaires ayant un nombre total de carbone allant de 35 à 70 comme le tétrapélargonate de pentaérythrityle (MM=697 g/mol),
   - les esters hydroxylés tels que le triisostéarate de polyglycérol-2 (MM=965 g/mol),
   - les esters aromatiques tels que le tridécyl trimellitate (MM=757 g/mol),
   - les esters d'alcool gras ou d'acides gras ramifiés en C₂₄-C₂₈ tels que ceux décrits dans la demande EP-A-0 955 039, et notamment le citrate de triisoarachidyle (MM=1033,76 g/mol), le tétraisononanoate de pentaérythrityle (MM=697 g/mol), le triisostéarate de glycéryle (MM=891 g/mol), le tri décyl-2 tétradécanoate de glycéryle (MM=1143 g/mol), le tétraisostéarate de pentaérythrityle (MM=1202 g/mol), le tétraisostéarate de polyglycéryle -2 (MM=1232 g/mol) ou encore le tétra décyl -2 tétradécanoate de pentaérythrityle (MM=1538 g/mol),
- les huiles siliconées telles que les silicones phénylées comme la BELSIL PDM 1000 de la société WACKER (MM=9000 g/mol),
- les huiles d'origine végétale telles que l'huile de sésame (820 g/mol),
- et leurs mélanges.

L'huile de masse molaire allant de 650 à 10000 g/mol utilisée dans la composition selon l'invention peut représenter de 1 à 99%, de préférence de 10 à 80%, et mieux de 5 à 70% du poids total de la composition.

Avantageusement, la composition de l'invention peut comprendre, en outre, au moins une matière colorante qui peut être choisie parmi les colorants solubles ou dispersibles dans la composition, les pigments, les nacres et leurs mélanges. Les colorants sont de préférence des colorants liposolubles, bien que les colorants hydrosolubles puissent être utilisés. Cette matière colorante peut représenter de 0,001 à 98 %, de préférence de 0,5 à 85% et mieux de 1 à 60 % du poids total de la composition.
Pour une composition sous forme de pâte ou coulée telle que les rouges à lèvres ou les produits de maquillage du corps, on utilise en général de 0,5 à 50% de matière colorante, de préférence de 2 à 40 % et mieux de 5 à 30%, par rapport au poids total de la composition.

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

De préférence, la composition de l'invention, comprend une phase particulaire avantageusement colorée pouvant représenter de 0,001 à 50 % du poids total de la composition, de préférence de 0,01 à 40 % et mieux de 0,05 à 30 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % (si présents) du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % (si présentes) du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré, les pigments goniochromatiques et par exemple les pigments multicouches interférentiels.

Les charges peuvent être présentes à raison de 0,001 à 35 % (si présentes) du poids total de la composition, de préférence 0,5 à 15 %. On peut notamment citer le talc, le mica, le kaolin, les poudres de Nylon® (Orgasol notamment) et de polyéthylène, les poudres de polytétrafluoroéthylène (Téflon®), l'amidon, le nitrure de bore, des microsphères de copolymères telles que l'Expancel® (Nobel Industrie), le Polytrap® (Dow Coming), le Polypore® L 200 (Chemdal Corporation) et les microbilles de résine de silicone (Tospearl ® de Toshiba, par exemple), la sillice.

La composition selon l'invention peut contenir au moins un composé non aqueux additionnel différent dudit ester aliphatique d'ester et de ladite huile de masse molaire allant de 650 à 10000 g/mol, choisi parmi d'autres huiles, les corps gras pâteux, les cires, les gommes, les résines et leurs mélanges.

En particulier, elle contient, en outre, au moins une cire. Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba; les cires synthétiques comme les cires de polyéthylène issues de la polymérisation ou copolymérisation de l'éthylène de masse moléculaire en poids compris entre 400 et 800 g/mol et leurs mélanges, les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.

Les gommes utilisables dans l'invention se présentent généralement sous forme solubilisée dans une huile, les polymères sont solides à température ambiante et les résines peuvent être liquides ou solides à température ambiante.

La nature et la quantité des gommes, corps pâteux ou cires sont fonction des propriétés mécaniques et des textures recherchées. A titre indicatif, la cire peut représenter de 0,01 à 50 %, de préférence de 2 à 40 %, et mieux de 5 à 30 % du poids total de la composition.

Les huiles additionnelles autres que les huiles de masse molaire allant de 650 à 10000 g/mol peuvent être des huiles hydrocarbonées et/ou siliconées et/ou fluorées. Ces huiles peuvent être d'origine animale, végétale, minérale ou synthétique. "Par huile hydrocarbonée", on entend une huile comportant principalement des atomes de carbone et d'hydrogène et éventuellement une ou plusieurs fonctions choisies parmi les fonctions hydroxyle, ester, éther, carboxylique. On utilise de préférence des huiles additionnelles d'origine végétale ou synthétique.

Les huiles additionnelles peuvent représenter de 0,01 à 90 % du poids total de la composition, de préférence de 0,1 à 60 % et mieux de 10 à 55 %.

La composition de l'invention peut comprendre, en outre, tout additif complémentaire usuellement utilisé dans le domaine concerné, tel que de l'eau, des antioxydants, des conservateurs, des neutralisants, des gélifiants lipophiles ou des composés non aqueux liquides, des gélifiants de phase aqueuse, des dispersants, des actifs cosmétiques. Ces additifs, à l'exception de l'eau qui peut représenter de 0 à 70 % et par exemple de 1 à 50 et mieux de 1 à 10 % du poids total de la composition, peuvent être présents dans la composition à raison de 0,0005 à 20% du poids total de la composition et mieux de 0,001 à 10%.

Comme actif cosmétique utilisable dans l'invention, on peut citer les vitamines A, E, C, B₃, F, les provitamines comme le D-panthénol, les actifs apaisants comme l'α-bisabolol, l'aloe vera, l'allantoïne, les extraits de plantes ou les huiles essentielles, les agents protecteurs ou restructurants comme les céramides, les actifs "fraîcheur" comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

Les applications des compositions selon l'invention sont multiples et concernent l'ensemble des produits cosmétiques colorés ou non et plus particulièrement les rouges à lèvres.

La composition de l'invention peut se présenter sous la forme de composition solide, compactée ou coulée notamment en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

Elle peut se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. De préférence, elle se présente sous forme de composition à phase continue huileuse et notamment anhydre ; dans ce cas, elle peut contenir une quantité d'eau inférieure à 5 %, de préférence inférieure à 1% en poids.

La composition selon l'invention peut se présenter sous la forme d'une composition, colorée ou non, de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent), produit de bronzage artificiel de la peau.

La composition de l'invention peut également se présenter sous la forme d'un produit de maquillage coloré de la peau, en particulier du visage comme un blush, un fard à joues ou à paupières, de maquillage du corps comme un produit de tatouage semi-permanent ou de maquillage des lèvres comme un rouge ou un brillant à lèvres, présentant éventuellement des propriétés de soin ou de traitement non thérapeutique, un produit de maquillage des phanères comme par exemple un vernis à ongles, un mascara, un eyeliner, un produit de coloration ou de soin des cheveux.

De préférence, la composition selon l'invention se présente sous forme d'un rouge à lèvres ou d'un brillant à lèvres.

Bien entendu la composition de l'invention doit être physiologiquement acceptable (en particulier cosmétiquement acceptable), à savoir non toxique et susceptible d'être appliquée sur la peau, les phanères ou les lèvres d'êtres humains.
Par « cosmétiquement acceptable », on entend agréable de goût, de toucher, d'aspect et/ou d'odeur, applicable plusieurs jours pendant plusieurs mois.

La composition selon l'invention peut être fabriquée par les procédés connus, généralement utilisés dans le domaine cosmétique.

Les exemples qui suivent ont pour but d'illustrer de manière non limitative l'objet de la présente invention. Les quantités sont données en pourcentage massique.

### Exemple 1 : Rouge à lèvres

| | |
|---|---|
| Ester d'huile de ricin d'acide succinique et d'acide isostéarique (commercialisé sous la référence Zénigloss par Zénitech) | 22 |
| Ethers de dodécanediol (22 mois) et de polyéthylène glycol (45 OE) (commercialisé sous la référence ELFACOS ST9 par Akzo Nobel) | 11 |
| Triglycéride d'acide 2-décyl-tetradécanoïque | 20 |
| Polyisobutène hydrogéné | 10 |
| Malate de di-isostéaryle | 11 |
| Polybutylène | 2,5 |
| Stéarate d'octacosanyle | 5 |
| Mélange de triglycérides d'acides laurique, myristique, palmitique, stearique (50/20/10/10) | 2 |
| Cire de polyéthylène | 5 |
| Hectorite modifiée par le chlorure de di-stéaryl di-méthyl ammonium | 3 |
| Pigments | qs |
| Conservateur | qs |
| Parfum | qs |

- La phase huileuse est réalisée en mélangeant le conservateur, toutes les huiles ainsi que le pâteux (éthers de dodécanediol (22 mols) et de polyéthylène glycol(45 OE)).
- Puis l'hectorite est broyée dans la phase huileuse à la tricylindre.
- Les pigments sont ensuite broyés dans le mélange hectorite et phase huileuse.
- On ajoute le mélange obtenu dans un poêlon avec les cires et on chauffe à 105°C pendant deux heures en homogénéisant à l'aide d'un appareil Raynerie.
- On ajoute enfin le parfum, on homogénéise 5 minutes puis on coule le mélange dans un moule à 42°C qui est placé à -20°C pendant 30 minutes. Puis on procède au démoulage des sticks.

La formule ci-dessus présente une bonne tenue de la brillance à 1 heure et ne migre pas à 1 heure. Elle possède en outre de bonnes propriétés en terme d'application (glissant), de confort, de brillance (à l'application et dans le temps) et de tenue de la couleur après épreuve.

### Exemple 2 comparatif :

La formule ci-dessus a été reproduite en remplaçant l'ester d'huile de ricin d'acide succinique et d'acide isostéarique par l'ester d'huile de ricin et d'acide benzoïque (commercialisé sous la référence Finsolv BCO 115 par Finetex).

La composition selon l'invention est plus nette aux contours et la tenue de sa couleur après épreuve est supérieure à celle contenant une huile brillante telle que l'ester d'huile de ricin et d'acide benzoïque. En outre son dépôt est plus épais.

## Revendications

1. Composition cosmétique de soin et/ou de maquillage des matières kératiniques comprenant un milieu cosmétiquement acceptable contenant i) au moins un polyester résultant de l'estérification d'au moins un triglycéride d'acide(s) carboxylique(s) hydroxylé(s) par a) un acide monocarboxylique aliphatique et par b) un acide dicarboxylique aliphatique, ii) au moins une huile de masse molaire allant de 650 à 10 000 g/mol, et iii) au moins une matière colorante.

2. Composition selon la revendication 1, **caractérisée en ce** ledit polyester est obtenu par
a) l'estérification par un acide monocarboxylique aliphatique d'une partie des fonctions hydroxyles d'un triglycéride d'acide(s) carboxylique(s) hydroxylé(s), puis par
b) l'estérification par un acide dicarboxylique des fonctions hydroxyles restantes dudit triglycéride d'acide(s) carboxylique(s) hydroxylé(s) estérifié par ledit acide monocarboxylique aliphatique.

3. Composition selon l'une des revendications précédentes, **caractérisée en ce que** le triglycéride d'acide(s) carboxylique(s) hydroxylé(s) est choisi parmi les triglycérides d'acide(s) carboxylique(s) hydroxylé(s) tels que ledit ou lesdits acides hydroxylés comprennent de 6 à 40 atomes de carbone, de préférence de 10 à 34 atomes de carbone et mieux de 12 à 28 atomes de carbone, de préférence de 16 à 20 atomes de carbone, de préférence 18 atomes de carbone.

4. Composition selon l'une quelconque des revendications précédentes, **caractérisée en ce que** l'acide ou les acides aliphatiques hydroxylés du triglycéride d'acide(s) carboxylique(s) hydroxylé(s) sont choisis parmi :
i) les acides monocarboxyliques aliphatiques mono hydroxylés linéaires saturés de formule :
(1) 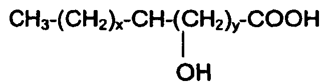 avec 3≤x+y≤37
ou (2)
HO-CH₂-(CH₂)ₓ-COOH
avec 4≤x≤38;
ii) les acides monocarboxyliques aliphatiques mono hydroxylés ramifiés saturés de formule : 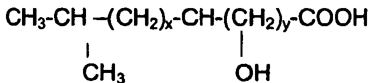
avec 1≤x+y≤35
Ou (3') l'acide 2-éthyl 3-hydroxy caprylique de formule: 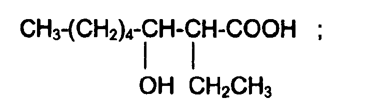
iii) les acides monocarboxyliques aliphatiques mono hydroxylés insaturés de formule :
(4) 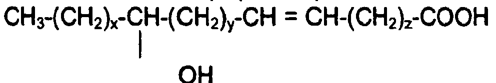 avec 1 ≤x+y+z≤ 35 ou
(5) 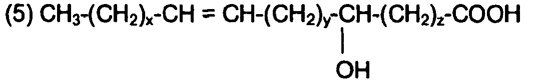 avec 1 ≤ x + y + z ≤ 35
ou (6) HOCH₂-(CH₂)ₓ-CH=CH-(CH₂)_{y}-COOHavec 2 ≤ x + y ≤ 36;
iv) les acides monocarboxyliques aliphatiques poly hydroxylés saturés de formule :
(7) 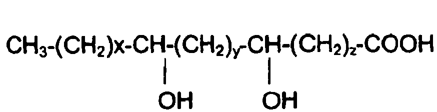 avec 2≤x+y+z≤36;
et les acides monocarboxyliques aliphatiques poly hydroxylés insaturés correspondants,
v) les polyacides aliphatiques mono hydroxylés saturés de formule :
(8) 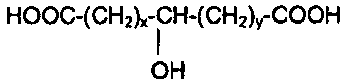 avec 3≤x+y≤37;
et les polyacides aliphatiques mono hydroxylés insaturés correspondants,
vi) les polyacides aliphatiques poly hydroxylés saturés ou insaturés; et leurs mélanges.

5. Composition selon l'une quelconque des revendications précédentes, **caractérisée en ce que** l'acide ou les acides hydroxylés aliphatiques sont choisis parmi :
- l'acide 12-hydroxy stéarique ; l'acide α-hydroxy octadécanoïque ; l'acide hydroxy 14-eicosènoïque
- l'acide leucinique ou l'acide 2-éthyl 3-hydroxy caprylique ;
- l'acide ricinoléïque ;
- l'acide 3-hydroxy 4-hexanoïque ou l'acide oxynervonique ;
- l'acide 16-hydroxy 6-hexadécènoïque ;
- l'acide 9, 10-dihydroxy octadécanoïque, l'acide 9, 12-dihydroxy octadécanoïque, l'acide aleuritique, l'acide 9, 10, 12-trihydroxy octadécanoïque, l'acide hexahydroxy octa-décanoique ou l'acide octahydroxy octadécanoïque ; et leurs mélanges.

6. Composition selon l'une des revendications précédentes, **caractérisée en ce que** le triglycéride est le triglycéride de l'acide ricinoléïque.

7. Composition selon l'une quelconque des revendications précédentes, **caractérisée en ce que** l'estérification dudit triglycéride d'acide(s) carboxylique(s) hydroxylé(s) est effectuée avec un acide monocarboxylique aliphatique comprenant de 6 à 40 atomes de carbones, de préférence de 10 à 34 atomes de carbone et mieux de 12 à 28 atomes de carbone, de préférence de 16 à 20 atomes de carbone, de préférence 18 atomes de carbone.

8. Composition selon la revendication précédente, **caractérisée en ce que** l'acide monocarboxylique aliphatique est un acide gras aliphatique saturé ou insaturé.

9. Composition selon la revendication précédente, **caractérisée en ce que** l'acide gras est l'acide isostéarique.

10. Composition l'une quelconque des revendications précédentes, **caractérisée en ce que** l'acide dicarboxylique aliphatique comprend de 3 à 10 atomes de carbones, de préférence de 3 à 6 atomes de carbone.

11. Composition selon la revendication précédente, **caractérisée en ce que** l'acide dicarboxylique aliphatique correspond à la formule HOOC-(CH₂)ₙ-COOH telle que n=1 à 4.

12. Composition selon la revendication précédente, **caractérisée en ce que** l'acide dicarboxylique aliphatique est l'acide succinique, de formule HOOC-(CH₂)ₙ-COOH telle que n = 2.

13. Composition selon la revendication 1, **caractérisée en ce que** le polyester répond à la formule
T₂O-(OC-D-CO-O-T₁-O)x-OC-D-CO-OT₂ (I)
dans laquelle T₂-O- provient du composé T₂-OH qui représente un triglycéride d'acide(s) carboxylique(s) hydroxylé(s) comprenant une seule fonction hydroxyle libre.
-O-T₁-O- provient du composé HO-T₁-OH qui représente un triglycéride d'acide(s) carboxylique(s) hydroxylé(s) comprenant deux fonctions hydroxyles libres.
-OC-D-CO- provient du composé HOOGD-COOH qui représente ledit acide dicarboxylique, et
x est compris entre 1 et 50, de préférence entre 1 et 10, de préférence encore entre 2 et 6.

14. Composition selon la revendication 13, **caractérisée en ce que** T₂-OH représente un triglycéride d'acide(s) carboxylique(s) hydroxylé(s), ledit tryglycéride étant estérifié par deux molécules d'un acide monocarboxylique aliphatique.

15. Composition selon la revendication 13, **caractérisée en ce que** HO-T₁-OH représente un triglycéride d'acide(s) carboxylique(s) hydroxylé(s), ledit tryglycéride étant estérifié par une molécule d'un acide monocarboxylique aliphatique.

16. Composition selon la revendication 13, **caractérisée en ce que** le polyester répond à la formule (1) dans laquelle
T₂O représente 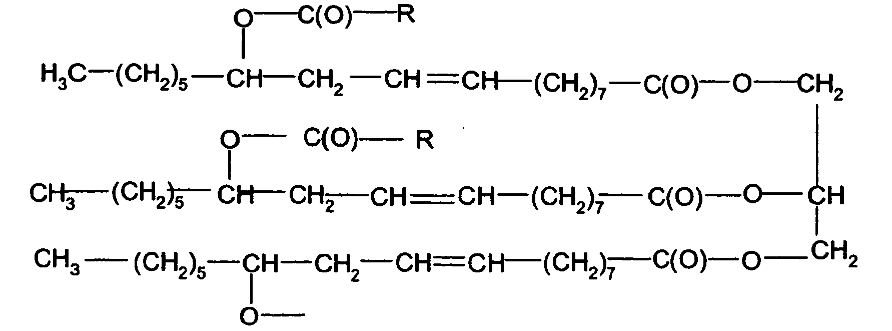
-OT₁O- représente 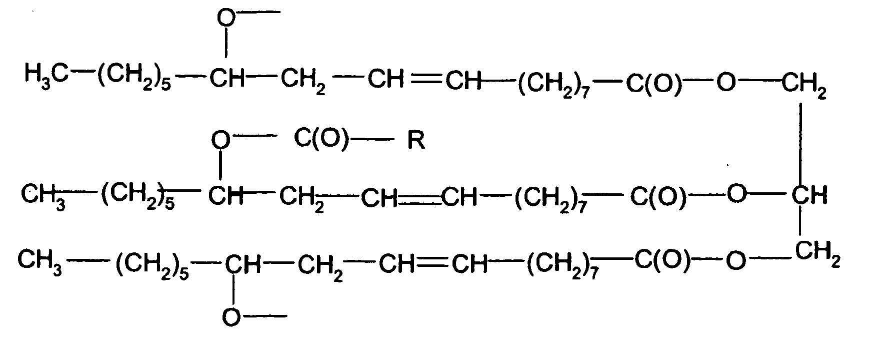
dans lesquelles R représente un groupe alkyle ou alkylène comprenant 5 à 33 atomes de carbone,

17. Composition selon la revendication précédente, **caractérisée en ce que** R représente un alkyle ayant 7 à 17 atomes de carbones.

18. Composition selon la revendication 16, **caractérisée en ce que** R représente un alkylène ayant de 11 à 21 atomes de carbones.

19. Composition selon l'une des revendications précédentes, **caractérisée en ce que** le polyester est liquide à température ambiante.

20. Composition selon la revendication précédente, **caractérisée en ce que** le polyester présente une viscosité supérieure à 500 cP (50 Pa.s) à 25°C, de préférence allant de 900 à 10 000 cP (90 à 1 000 Pa.s) et mieux de 950 à 5 000 cP (95 à 500 Pa.s) et/ou un indice de réfraction ≥ 1,48.

21. Composition selon l'une des revendications précédentes, **caractérisée en ce que** le polyester est présent en une quantité suffisante pour conférer à la composition des propriétés de glissant, de brillance, de stabilité et/ou de tenue de la couleur dans le temps, de tenue de la brillance dans le temps, de confort, de non migration et/ou de netteté des contours du dépôt de ladite composition.

22. Composition selon l'une des revendications précédentes, **caractérisée en ce que** le polyester est présent en une quantité allant de 10 à 40 %, de préférence de 15 à 25 % et mieux de 20 à 25 % du poids total de la composition.

23. Composition selon l'une des revendications précédentes, **caractérisée en ce que** l'huile de masse molaire élevée a une masse molaire allant de 750 à 7500 g/mol.

24. Composition selon l'une des revendications précédentes, **caractérisée en ce que** l'huile de masse molaire élevée est choisie parmi :
- les polymères lipophiles
- les esters d'acides gras linéaires ayant un nombre total de carbones allant de 35 à 70
- les esters hydroxylés,
- les esters aromatiques,
- les esters d'alcool gras ou d'acides gras ramifiés en C₂₄-C₂₈,
- les huiles siliconées,
- les huiles d'origine végétale,
et leurs mélanges.

25. Composition selon l'une des revendications précédentes, **caractérisée en ce que** l'huile de masse molaire élevée est choisie parmi les polybutylènes, les polyisobutylènes hydrogénés, les polydécènes, les polydécènes hydrogénés, les copolymères de la vinylpyrrolidone tel que le PVP / héxadécène copolymère, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de glycéryle, le tri décyl -2 tétradécanoate de glycéryle, le tétraisostéarate de pentaérythrityle, le tétraisostéarate de polyglycéryle-2, le tétra décyl-2 tétradécanoate de pentaérythrityle, les silicones phénylées, l'huile de sésame, et leurs mélanges.

26. Composition selon l'une des revendications précédentes, **caractérisée en ce que** l'huile de masse molaire élevée représente de 1 à 99%, de préférence de 10 à 80% et mieux de 5 à 70 % du poids total de la composition.

27. Composition selon l'une des revendications précédentes, **caractérisée en ce qu**'elle se présente sous forme d'un rouge à lèvres ou d'un brillant à lèvres, d'un fard à joues ou à paupières, d'un mascara, d'un eye-liner, d'un vernis à ongles, d'un produit de bronzage artificiel de la peau, d'un produit de coloration ou de soin des cheveux.

28. Composition selon la revendication précédente, **caractérisée en ce que** la matière colorante est choisie parmi les colorants solubles ou dispersibles dans la composition, les pigments, les nacres et leurs mélanges.

29. Composition selon l'une des revendications précédentes, **caractérisée en ce qu'**elle contient, en outre, au moins une cire choisie parmi les cires de polyéthylène de poids moléculaire compris entre 400 et 800 g/mol.

30. Composition selon l'une des revendications précédentes, **caractérisée en ce qu**'elle se présente sous forme coulée ou compactée.

31. Composition selon l'une des revendications précédentes, **caractérisée en ce qu**'elle se présente sous forme anhydre.

32. Composition selon l'une des revendications précédentes, **caractérisée en ce qu**'elle se présente sous forme d'un rouge à lèvres ou d'un brillant à lèvres.

33. Procédé cosmétique pour conférer à un film de composition cosmétique de bonnes propriétés à l'application, de bonnes propriétés d'étalement, de tenue de la couleur après épreuves, de confort (non tiraillement, non dessèchement), de non migration et/ou dont les contours du dépôt sur les matières kératiniques sont nets et/ou dont l'intensité de la couleur est améliorée, ledit procédé consistant à introduire dans ladite composition i) au moins un polyester résultant de l'estérification d'au moins un triglycéride d'acide(s) carboxylique(s) hydroxylé(s) par un acide monocarboxylique aliphatique et par un acide dicarboxylique aliphatique, ii) au moins une huile de masse molaire élevée allant de 650 à 10000 g/mol, et iii) au moins une matière colorante.

34. Utilisation de l'association i) d'au moins un polyester résultant de l'estérification d'au moins un triglycéride d'acide(s) carboxylique(s) hydroxylé(s) par un acide monocarboxylique aliphatique et par un acide dicarboxylique aliphatique et ii) d'au moins une huile de masse molaire élevée allant de 650 à 10000 g/mol dans une composition physiologiquement acceptable contenant une matière colorante, pour conférer à ladite composition de bonnes propriétés à l'application, de bonnes propriétés d'étalement, de tenue de la couleur après épreuves, de confort (non tiraillement, non dessèchement), de non migration et/ou d'intensité de la couleur améliorée et/ou de tenue de la couleur après épreuves améliorée.

## Claims

1. Cosmetic care and/or make up composition for keratin materials comprising a cosmetically acceptable medium comprising i) at least one polyester resulting from the esterification of at least one triglyceride of hydroxylated carboxylic acid(s) by a) an aliphatic monocarboxylic acid and by b) an aliphatic dicarboxylic acid, ii) at least one oil of molar mass ranging from 650 to 10 000 g/mol, and iii) at least one colorant material.

2. Composition according to Claim 1, **characterized in that** said polyester is obtained by:
a) esterification by an aliphatic monocarboxylic acid of one portion of the hydroxyl functional groups of a triglyceride of hydroxylated carboxylic acid(s); then by
b) esterification by a dicarboxylic acid of the remaining hydroxyl functional groups of said triglyceride of hydroxylated carboxylic acid(s) esterified by said aliphatic monocarboxylic acid.

3. Composition according to either of the preceding claims, **characterized in that** the triglyceride of hydroxylated carboxylic, acid(s) is chosen from the triglycerides of hydroxylated carboxylic acid(s) such as said hydroxylated acid(s) comprising from 6 to 40 carbon atoms, preferably from 10 to 34 carbon atoms and better still from 12 to 28 carbon atoms, preferably from 16 to 20 carbon atoms and preferably 18 carbon atoms.

4. Composition according to any one of the preceding claims, **characterized in that** the hydroxylated aliphatic acid or acids of the triglyceride of hydroxylated carboxylic acid(s) are chosen from:
i) saturated linear monohydroxylated aliphatic monocarboxylic acids of formula:
(1) 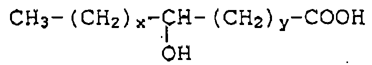 with 3 ≤ x+y ≤ 37
or (2)
HO-CH₂-(CH₂)ₓ-COOH
with 4 ≤ x ≤ 38;
ii) saturated branched monohydroxylated aliphatic monocarboxylic acids of formula:
(3) 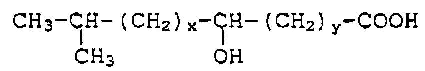 with 1 ≤ x+y ≤ 35
or (3') 2-ethyl-3-hydroxycaprylic acid of formula: 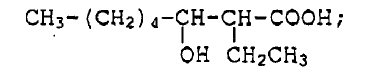
iii) unsaturated monohydroxylated aliphatic monocarboxylic acids of formula:
(4) 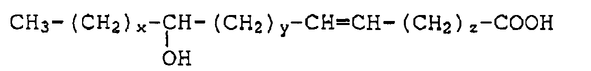 with 1 ≤ x+y+z ≤ 35
or
(5) 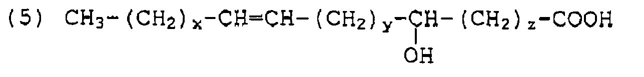 with 1 ≤ x+y+z ≤ 35
or (6)
HOCH₂-(CH₂)ₓ-CH=CH-(CH₂)_{y}-COOH
with 2 ≤ x+y ≤ 36;
iv) saturated polyhydroxylated aliphatic monocarboxylic acids of formula:
(7) 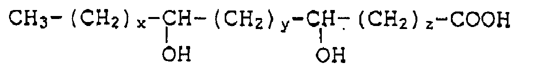 with 2 ≤ x+y+z ≤ 36;
and the corresponding unsaturated polyhydroxylated aliphatic monocarboxylic acids;
v) saturated monohydroxylated aliphatic polyacids of formula:
(8) 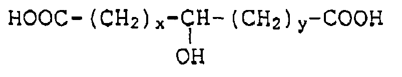 with 3 ≤ x+y ≤ 37;
and the corresponding unsaturated monohydroxylated aliphatic polyacids;
vi) saturated or unsaturated polyhydroxylated aliphatic polyacids;
and mixtures thereof.

5. Composition according to any one of the preceding claims, **characterized in that** the aliphatic hydroxylated acid or acids are chosen from:
- 12-hydroxystearic acid; α-hydroxyoctadecanoic acid; hydroxy-14-eicosenoic acid;
- leucinic acid or 2-ethyl-3-hydroxycaprylic acid;
- ricinoleic acid;
- 3-hydroxy-4-hexanoic acid or oxynervonic acid;
- 16-hydroxy-6-hexadecenoic acid;
- 9,10-dihydroxyoctadecanoic acid, 9,12-dihydroxyoctadecanoic acid, aleuritic acid, 9,10,12-trihydroxyoctadecanoic acid, hexahydroxyoctadecanoic acid or octahydroxyoctadecanoic acid; and mixtures thereof.

6. Composition according to any one of the preceding claims, **characterized in that** the triglyceride is the triglyceride of ricinoleic acid.

7. Composition according to any one of the preceding claims, **characterized in that** the esterification of said triglyceride of hydroxylated carboxylic acid(s) is carried out with an aliphatic monocarboxylic acid comprising from 6 to 40 carbon atoms, preferably from 10 to 34 carbon atoms and better still from 12 to 28 carbon atoms, preferably from 16 to 20 carbon atoms and preferably 18 carbon atoms.

8. Composition according to the preceding claim, **characterized in that** the aliphatic monocarboxylic acid is a saturated or unsaturated aliphatic fatty acid.

9. Composition according to the preceding claim, **characterized in that** the fatty acid is isostearic acid.

10. Composition according to any one of the preceding claims, **characterized in that** the aliphatic dicarboxylic acid comprises from 3 to 10 carbon atoms, preferably from 3 to 6 carbon atoms.

11. Composition according to the preceding claim, **characterized in that** the aliphatic dicarboxylic acid corresponds to the formula HOOC-(CH₂)ₙ-COOH such that n = 1 to 4.

12. Composition according to the preceding claim, **characterized in that** the aliphatic dicarboxylic acid is succinic acid, of formula HOOC-(CH₂)ₙ-COOH such that n = 2.

13. Composition according to Claim 1, **characterized in that** the polyester fits the formula:
T₂O-(OC-O-CO-O-T₁-O)ₓ-OC-D-CO-OT₂ (I)
in which:
T₂-O- derives from the compound T₂-OH that represents a triglyceride of hydroxylated carboxylic acid(s) comprising a single free hydroxyl functional group;
-O-T₁-O- derives from the compound HO-T₁-OH that represents a triglyceride of hydroxylated carboxylic acid(s) comprising two free hydroxyl functional groups;
-OC-D-CO- derives from the compound HOOC-D-COOH that represents said dicarboxylic acid; and
x is between 1 and 50, preferably between 1 and 10, more preferably between 2 and 6.

14. Composition according to Claim 13, **characterized in that** T₂-OH represents a triglyceride of hydroxylated carboxylic acid(s), said triglyceride being esterified by two molecules of an aliphatic monocarboxylic acid.

15. Composition according to Claim 13, **characterized in that** HO-T₁-OH represents a triglyceride of hydroxylated carboxylic acid(s), said triglyceride being esterified by one molecule of an aliphatic monocarboxylic acid.

16. Composition according to Claim 13, **characterized in that** the polyester fits the formula (I) in which T₂O represents: 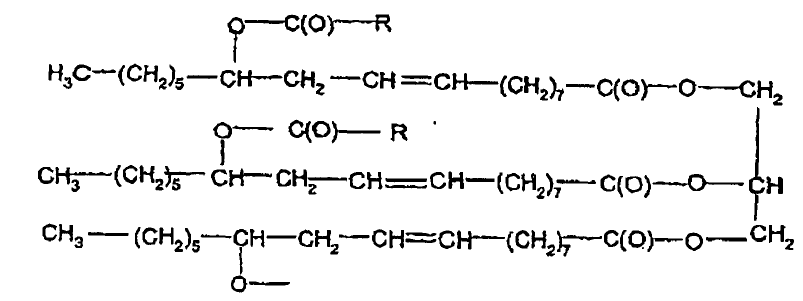 and -O-T₁-O- represents: 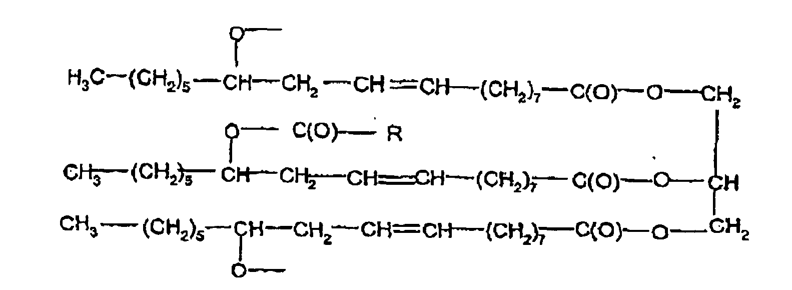 in which R represents an alkyl or alkylene group comprising 5 to 33 carbon atoms.

17. Composition according to the preceding claim, **characterized in that** R represents an alkyl group having from 7 to 17 carbon atoms.

18. Composition according to Claim 16, **characterized in that** R represents an alkylene group having from 11 to 21 carbon atoms.

19. Composition according to one of the preceding claims, **characterized in that** the polyester is liquid at room temperature.

20. Composition according to the preceding claim, **characterized in that** the polyester has a viscosity greater than 500 cP (50 Pa.s) at 25°C, preferably ranging from 900 to 10000 cP (90 to 1000 Pa.s) and better still from 950 to 5000 cP (95 to 500 Pa.s) and/or a refractive index ≥ 1.48.

21. Composition according to one of the preceding claims, **characterized in that** the polyester is present in a sufficient quantity to confer on the composition the properties of slip, gloss, stability and/or retention of the colour over time, gloss retention over time, comfort, migration resistance and/or outline definition of the deposited layer of said composition.

22. Composition according to one of the preceding claims, **characterized in that** the polyester is present in a quantity ranging from 10 to 40%, preferably from 15 to 25% and better still from 20 to 25% of the total weight of the composition.

23. Composition according to one of the preceding claims, **characterized in that** the oil of high molar mass has a molar mass ranging from 750 to 7500 g/mol.

24. Composition according to one of the preceding claims, **characterized in that** the oil of high molar mass is chosen from:
- lipophilic polymers;
- linear fatty acid esters having a total number of carbon atoms ranging from 35 to 70;
- hydroxylated esters;
- aromatic esters;
- C₂₄-C₂₈ branched fatty alcohol or fatty acid esters;
- silicone oils;
- oils of plant origin;
and mixtures thereof.

25. Composition according to one of the preceding claims, **characterized in that** the oil of high molar mass is chosen from: polybutylenes, hydrogenated polyisobutylenes, polydecenes, hydrogenated polydecenes, vinylpyrrolidone copolymers such as the PVP/hexadecene copolymer, pentaerythrityl tretrapelargonate, polyglyceryl-2 triisostereate, tridecyl trimellicate, triisoarachidyl citrate, pentaerythrityl tetraisononanoate, glyceryl triisostereate, glyceryl tri(2-decyltetradecanoate), pentaerythrityl tetraisostereate, polyglyceryl-2 tetraisostereate, pentaerythrityl tetra(2-decyltetradecanoate), phenyl silicones, sesame oil, and mixtures thereof.

26. Composition according to one of the preceding claims, **characterized in that** the oil of high molar mass represents from 1 to 99%, preferably from 10 to 80% and better still from 5 to 70% of the total weight of the composition.

27. Composition according to one of the preceding claims, **characterized in that** it is in the form of a lipstick or lip gloss, a face powder or eye shadow, a mascara, an eyeliner, a nail varnish, an artificial tanning product for the skin, a hair care or colouring product.

28. Composition according to the preceding claim, **characterized in that** the colouring material is chosen from dyes that are soluble or dispersible in the composition, pigments, nacres and mixtures thereof.

29. Composition according to one of the preceding claims, **characterized in that** it comprises, moreover, at least one wax chosen from polyethylene waxes of molecular weight between 400 and 800 g/mol.

30. Composition according to one of the preceding claims, **characterized in that** it is in cast or compacted form.

31. Composition according to one of the preceding claims, **characterized in that** it is in anhydrous form.

32. Composition according to one of the preceding claims, **characterized in that** it is in the form of a lipstick or lip gloss.

33. Cosmetic method for conferring on a film of cosmetic composition good application properties, good properties of spreading, of colour retention after trials, of comfort (no tightening, no dryness), of migration-resistance and/or where the outlines, when deposited on keratin materials, are sharply defined and/or where the colour intensity is improved, said method consisting in introducing into said composition i) at least one polyester resulting from the esterification of at least one triglyceride of hydroxylated carboxylic acid(s) by an aliphatic monocarboxylic acid and by an aliphatic dicarboxylic acid, ii) at least one oil of high molar mass ranging from 650 to 10 000 g/mol, and iii) at least one colouring material.

34. Use of the combination of i) at least one polyester resulting from the esterification of at least one triglyceride of hydroxylated carboxylic acid(s) by an aliphatic monocarboxylic acid and by an aliphatic dicarboxylic acid and ii) at least one oil of high molar mass ranging from 650 to 10 000 g/mol in a physiologically acceptable composition comprising a colouring material, to confer on said composition good application properties, good properties of spreading, of colour retention after trials, of comfort (no tightening, no dryness), of migration-resistance and/or of improved colour intensity and/or of improved colour retention after trials.

## Patentansprüche

1. Kosmetische Zusammensetzung für die Pflege und/oder zum Schminken von Keratinsubstanzen, die ein kosmetisch akzeptables Medium enthält, das i) mindestens einen Polyester, der bei der Veresterung mindestens eines Triglycerids von hydroxylierter (hydroxylierten) Carbonsäure(n) mit a) einer aliphatischen Monocarbonsäure und b) einer aliphatischen Dicarbonsäure gebildet wird, ii) mindestens ein Öl mit einer Molmasse von 650 bis 10 000 g/mol und iii) mindestens ein Farbmittel enthält.

2. Zusammensetzung nach Anspruch 1, **dadurch gekennzeichnet, dass** der Polyester hergestellt wird durch
a) Veresterung eines Teils der Hydroxyfunktionen eines Triglycerids von hydroxylierter (hydroxylierten) Carbonsäure(n) mit einer aliphatischen Monocarbonsäure und anschließend
b) Veresterung der restlichen Hydroxyfunktionen des Triglycerids von hydroxylierter (hydroxylierten) Carbonsäure(n), das mit der aliphatischen Monocarbonsäure verestert wurde, mit einer Dicarbonsäure.

3. Zusammensetzung nach einem der vorhergehenden Ansprüche, **dadurch gekennzeichnet, dass** das Triglycerid von hydroxylierter (hydroxylierten) Carbonsäure(n) unter den Triglyceriden von hydroxylierter (hydroxylierten) Carbonsäure(n) ausgewählt ist, die so vorliegen, dass die hydroxylierte(n) Säure(n) 6 bis 40 Kohlenstoffatome, vorzugsweise 10 bis 34 Kohlenstoffatome, besser 12 bis 28 Kohlenstoffatome, bevorzugt 16 bis 20 Kohlenstoffatome und vorzugsweise 18 Kohlenstoffatome aufweisen.

4. Zusammensetzung nach einem der vorhergehenden Ansprüche, **dadurch gekennzeichnet, dass** die hydroxylierte(n) aliphatische(n) Säure(n) des Triglycerids von hydroxylierter (hydroxylierten) Carbonsäure(n) ausgewählt sind unter:
i) gesättigten, geradkettigen, monohydroxylierten, aliphatischen Monocarbonsäuren der Formel:
(1) 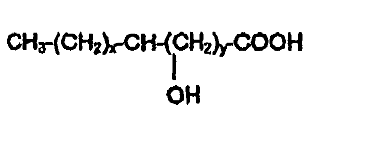 mit 3 ≤x+y≤37 oder
(2)
HO-CH₂-(CH₂)ₓ-COOH
mit 4 ≤ x ≤ 38;
ii) gesättigten, verzweigten, monohydroxylierten, aliphatischen Monocarbonsäuren der Formel:
(3) 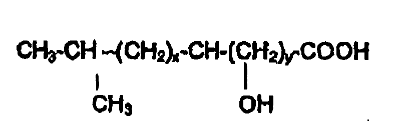 mit 1 ≤ x + y ≤ 35 oder
(3') 2-Ethyl-3-hydroxycaprylsäure der Formel: 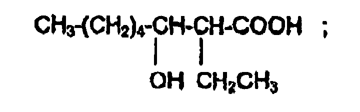
(iii) ungesättigten, monohydroxylirten, aliphatischen Monocarbonsäuren der Formel:
(4) 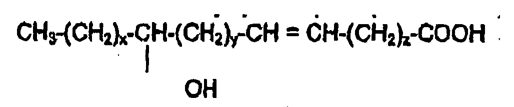 mit 1≤x+y+z≤35 oder
(5) 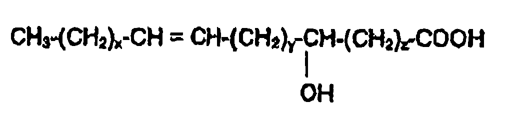 mit 1≤x+y+z≤35 oder
(6)
HOCH₂-(CH₂)ₓ-CH = CH-(CH₂)_{y}-COOH
mit 2≤x+y+z≤36
iv) gesättigten, mehrfach hydroxylierten, aliphatischen Monocarbonsäuren der Formel:
(7) 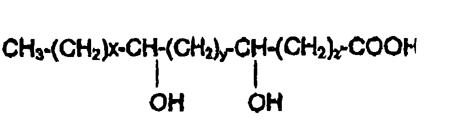 mit 2≤x+y+z≤36;
und den entsprechenden ungesättigten, mehrfach hydroxylierten, aliphatischen Monocarbonsäuren,
v) gesättigten, monohydroxylierten, aliphatischen Polysäuren der Formel: 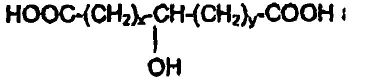
mit 3≤x+y≤37;
und den entsprechenden ungesättigten, monohydroxylierten, aliphatischen Polysäuren,
(vi) gesättigten oder ungesättigten, mehrfach hydroxylierten, aliphatischen Polysäuren;
und deren Gemischen.

5. Zusammensetzung nach einem der vorhergehenden Ansprüche, **dadurch gekennzeichnet, dass** die hydroxylierte(n) aliphatische(n) Säure(n) ausgewählt sind unter:
- 12-Hydroxystearinsäure; α-Hydroxyoctadecansäure; 14-Hydroxyeicosensäure;
- Leucinsäure oder 2-Ethyl-3-hydroxycaprylsäure;
- Ricinolsäure;
- 3-Hydroxy-4-hexansäure oder Oxynervonsäure;
- 16-Hydroxy-6-hexadecensäure;
- 9,10-Dihydroxyoctadecansäure, 9,12-Dihydroxyoctadecansäure, Aleuritinsäure, 9,10,12-Trihydroxyoctadecansäure, Hexahydroxyoctadecansäure oder Octahydroxyoctadecansäure;
und deren Gemischen.

6. Zusammensetzung nach einem der vorhergehenden Ansprüche, **dadurch gekennzeichnet, dass** das Triglycerid das Triglycerid von Ricinolsäure ist.

7. Zusammensetzung nach einem der vorhergehenden Ansprüche, **dadurch gekennzeichnet, dass** die Veresterung des Triglycerids von hydroxylierter (hydroxylierten) Carbonsäure(n) mit einer aliphatischen Monocarbonsäure durchgeführt wird, die 6 bis 40 Kohlenstoffatome, vorzugsweise 10 bis 34 Kohlenstoffatome, besser 12 bis 28 Kohlenstoffatome, bevorzugt 16 bis 20 Kohlenstoffatome und vorzugsweise 18 Kohlenstoffatome aufweist.

8. Zusammensetzung nach dem vorhergehenden Anspruch, **dadurch gekennzeichnet, dass** die aliphatische Monocarbonsäure eine gesättigte oder ungesättigte aliphatische Fettsäure ist.

9. Zusammensetzung nach dem vorhergehenden Anspruch, **dadurch gekennzeichnet, dass** es sich bei der Fettsäure um Isostearinsäure handelt,

10. Zusammensetzung nach einem der vorhergehenden Ansprüche, **dadurch gekennzeichnet, dass** die aliphatische Dicarbonsäure 3 bis 10 Kohlenstoffatome und vorzugsweise 3 bis 6 Kohlenstoffatome aufweist.

11. Zusammensetzung nach dem vorhergehenden Anspruch, **dadurch gekennzeichnet, dass** die aliphatische Dicarbonsäure der Formel HOOC-(CH₂)ₙ-COOH entspricht, mit n = 1 bis 4.

12. Zusammensetzung nach dem vorhergehenden Anspruch, **dadurch gekennzeichnet, dass** die aliphatische Dicarbonsäure die Bernsteinsäure der Formel HOOC-(CH₂)ₙ-COOH ist, mit n = 2.

13. Zusammensetzung nach Anspruch 1, **dadurch gekennzeichnet, dass** der Polyester der folgenden Formel entspricht:
T₂O-(OC-D-CO-O-T₁-O)x-OC-D-CO-OT₂ (I),
worin T₂-O- von der Verbindung T₂-OH stammt, die ein Triglycerid einer oder mehrerer hydroxylierter Fettsäuren mit einer einzigen freien Hydroxyfunktion bedeutet,
-O-T₁-O- von der Verbindung HO-T₁-OH stammt, die ein Triglycerid von hydroxylierter (hydroxylierten) Carbonsäure(n) mit zwei freien Hydroxyfunktionen bedeutet,
-OC-D-CO- von der Verbindung HOOC-D-COOH stammt, die die Dicarbonsäure bedeutet, und
x im Bereich von 1 bis 50, vorzugsweise 1 bis 10 und noch bevorzugter 2 bis 6 liegt.

14. Zusammensetzung nach Anspruch 13, **dadurch gekennzeichnet, dass** T₂-OH ein Triglycerid von hydroxylierter (hydroxylierten) Carbonsäure(n) bedeutet, wobei das Triglycerid mit zwei Molekülen einer aliphatischen Monocarbonsäure verestert ist.

15. Zusammensetzung nach Anspruch 13, **dadurch gekennzeichnet, dass** HO-T₁-OH ein Triglycerid von hydroxylierter (hydroxylierten) Carbonsäure(n) bedeutet, wobei das Triglycerid mit einem Molekül einer aliphatischen Monocarbonsäure verestert ist.

16. Zusammensetzung nach Anspruch 13, **dadurch gekennzeichnet, dass** der Polyester der Formel (I) entspricht, worin
T₂O bedeutet 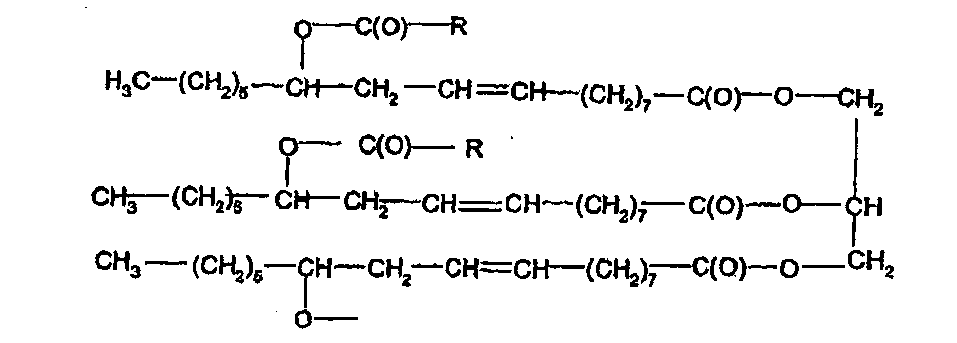
-OT₁O- bedeutet 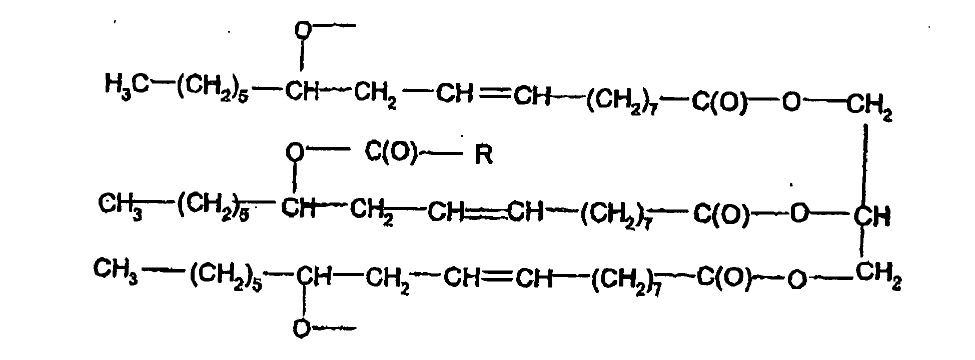
wobei R eine Alkyl- oder Alkylengruppe mit 5 bis 33 Kohlenstoffatomen ist.

17. Zusammensetzung nach dem vorhergehenden Anspruch, **dadurch gekennzeichnet, dass** R eine Alkylgruppe mit 7 bis 17 Kohlenstoffatomen ist.

18. Zusammensetzung nach Anspruch 16, **dadurch gekennzeichnet, dass** R eine Alkylengruppe mit 11 bis 21 Kohleristoffatomen ist.

19. Zusammensetzung nach einem der vorhergehenden Ansprüche, **dadurch gekennzeichnet, dass** der Polyester bei Umgebungstemperatur flüssig ist.

20. Zusammensetzung nach dem vorhergehenden Anspruch, **dadurch gekennzeichnet, dass** der Polyester bei 25 °C eine Viskosität über 500 cP (50 Pa·s), vorzugsweise im Bereich von 900 bis 10 000 cP (90 bis 1 000 Pa.s) und besser 950 bis 5 000 cP (95 bis 500 Pa·s) aufweist und/oder einen Brechungsindex ≥ 1,48 aufweist.

21. Zusammensetzung nach einem der vorhergehenden Ansprüche, **dadurch gekennzeichnet, dass** der Polyester in einer Menge vorliegt, die ausreichend ist, damit die Zusammensetzung die Eigenschaften Gleiten, Glanz, Stabilität und/oder Langzeitfarbbeständigkeit, Langzeitglanzbeständigkeit, Komfort, Nichtmigration und/oder Klarheit der Konturen der Abscheidung der Zusammensetzung erhält.

22. Zusammensetzung nach einem der vorhergehenden Ansprüche, **dadurch gekennzeichnet, dass** der Polyester in einer Menge von 10 bis 40 %, vorzugsweise 15 bis 25 % und besser 20 bis 25 % des Gesamtgewichts der Zusammensetzung enthalten ist.

23. Zusammensetzung nach einem der vorhergehenden Ansprüche, **dadurch gekennzeichnet, dass** das Öl mit hoher Molmasse eine Molmasse von 750 bis 7000 g/mol aufweist.

24. Zusammensetzung nach einem der vorhergehenden Ansprüche, **dadurch gekennzeichnet, dass** das Öl mit hoher Molmasse ausgewählt ist unter:
- lipophilen Polymeren,
- linearen Fettsäureestern mit einer Gesamtzahl der Kohlenstoffatome von 35 bis 70,
- hydroxylierten Estern,
- aromatischen Estern,
- Estern von Fettalkoholen oder Fettsäuren, die verzweigt sind und 24 bis 28 Kohlenstoffatome aufweisen,
- siliconierten Ölen,
- Ölen, pflanzlicher Herkunft,
und deren Gemischen.

25. Zusammensetzung nach einem der vorhergehenden Ansprüche, **dadurch gekennzeichnet, dass** das Öl mit einer hohen Molmasse unter den Polybutylenen, hydrierten Polyisobutylenen, Polydecenen, hydrierten Polydecenen, Copolymeren von Vinylpyrrolidon, wie dem PVP/Hexadecen-Copolymer, Pentaerythrityltetrapelargonat, Diglyceryltriisostearat, Tridecyltrimellitat, Triisoarachidylcitrat, Pentaerythrityltetraisononanoat, Glyeryltriisostearat, Glyeryltri-2-decyltetradecanoat, Pentaerythrityltetraisostearat, Diglyceryltetraisostearat, Pentaerythrityltetra-2-decyltetradecanoat, phenylierten Siliconen, Sesamöl und deren Gemischen ausgewählt ist.

26. Zusammensetzung nach einem der vorhergehenden Ansprüche, **dadurch gekennzeichnet, dass** das Öl mit einer hohen Molmasse 1 bis 99 %, vorzugsweise 10 bis 80 % und besser 5 bis 70 % des Gesamtgewichts der Zusammensetzung ausmacht.

27. Zusammensetzung nach einem der vorhergehenden Ansprüche, **dadurch gekennzeichnet, dass** sie als Lippenstift oder Lipgloss, Lidschatten, Wangenrouge, Mascara, Eyeliner, Nagellack, Produkt zur künstlichen Braunfärbung der Haut, Produkt zum Färben oder für die Pflege der Haare vorliegt.

28. Zusammensetzung nach dem vorhergehenden Anspruch, **dadurch gekennzeichnet, dass** das Farbmittel unter den in der Zusammensetzung löslichen oder dispergierbaren Farbstoffen, Pigmenten, Perlglanzpigmenten und deren Gemischen ausgewählt ist.

29. Zusammensetzung nach einem der vorhergehenden Ansprüche, **dadurch gekennzeichnet, dass** sie ferner mindestens ein Wachs enthält, das unter den Polyethylenwachsen mit einer Molmasse von 400 bis 800 g/mol ausgewählt ist.

30. Zusammensetzung nach einem der vorhergehenden Ansprüche, **dadurch gekennzeichnet, dass** sie in gegossener oder kompaktierter Form vorliegt.

31. Zusammensetzung nach einem der vorhergehenden Ansprüche, **dadurch gekennzeichnet, dass** sie in wasserfreier Form vorliegt.

32. Zusammensetzung nach einem der vorhergehenden Ansprüche, **dadurch gekennzeichnet, dass** sie als Lippenstift oder Lipgloss vorliegt.

33. Kosmetisches Verfahren, um einem Film einer kosmetischen Zusammensetzung gute Eigenschaften beim Aufbringen, gute Eigenschaften beim Verteilen, Farbbeständigkeit nach Druckproben, Komfort (kein Ziehen, kein Austrocknen), Nichtmigration zu verleihen und/oder damit die Konturen der Abscheidung auf den Keratinsubstanzen deutlich sind und/oder die Farbintensität besser ist, wobei das Verfahren darin besteht, in die Zusammensetzung i) mindestens einen Polyester, der bei der Veresterung mindestens eines Triglycerids von hydroxylierter (hydroxylierten) Carbonsäure(n) mit einer aliphatischen Monocarbonsäure und mit einer aliphatischen Dicarbonsäure gebildet wird, ii) mindestens ein Öl mit einer hohen Molmasse von 650 bis 10 000 g/mol und iii) mindestens ein Farbmittel einzuarbeiten.

34. Verwendung der Kombination i) mindestens eines Polyesters, der bei der Veresterung mindestens eines Triglycerids von hydroxylierter (hydroxylierten) Carbonsäure(n) mit einer aliphatischen Monocarbonsäure und mit einer aliphatischen Dicarbonsäure gebildet wird, und ii) mindestens eines Öls mit hoher Molmasse von 650 bis 10 000 g/mol in einer physiologisch akzeptablen Zusammensetzung, die ein Farbmittel enthält, um der Zusammensetzung gute Eigenschaften beim Aufbringen, gute Eigenschaften beim Verteilen, Farbbeständigkeit nach Druckproben, Komfort (kein Ziehen, kein Austrocknen), Nichtmigration und/oder eine bessere Farbintensität und/oder eine bessere Farbbeständigkeit nach Druckproben zu geben.
